# EUROPEAN PATENT SPECIFICATION

(11) **EP 1 109 579 B1**
(45) Date of publication and mention of the grant of the patent: **07.12.2005**
(21) Application number: 99946087.6
(22) Date of filing: 01.09.1999
(51) Int. Cl.: A61K 39/395, A61K 38/48, A61K 38/49

(54) **USE OF TGF-BETA INHIBITORS FOR TREATING CEREBRAL DISORDERS**
VERWENDUNG VON TGF-BETA INHIBITOREN ZUR BEHANDLUNG VON CEREBRALEN KRANKHEITEN
UTILISATION D'INHIBITEURS DU FACTEUR DE CROISSANCE TRANSFORMANT BETA POUR TRAITER DES TROUBLES CEREBRAUX

(30) Priority: 03.09.1998 EP 98116692; 16.03.1999 EP 99104033
(43) Date of publication of application: 27.06.2001
(73) Proprietor: BIOPHARM GESELLSCHAFT ZUR BIOTECHNOLOGISCHEN ENTWICKLUNG UND ZUM VERTRIEB VON PHARMAKA mbh, 69115 Heidelberg (DE)
(72) Inventor: KRIEGLSTEIN, Kerstin, D-69118 Heidelberg (DE)
(74) Representative: Müller-Boré & Partner Patentanwälte
(86) International application number: PCT/EP1999/006433
(87) International publication number: WO 2000/013705

(56) References cited:
- EP-A- 0 181 267
- WO-A-93/19783
- WO-A-95/10611
- US-A- 5 399 487
- K. FLANDERS ET AL.: "Transforming growth factor-betas in neurodegenerative disease." PROGRESS IN NEUROBIOLOGY, vol. 54, no. 1, January 1998 (1998-01), pages 71-85, XP000857248 Oxford, GB
- K. KRIEGLSTEIN ET AL.: "Distinct modulatory actions of TGF-beta and LIF on neurotrophin-mediated survival of developing sensory neurons." NEUROCHEMICAL RESEARCH, vol. 21, no. 7, July 1996 (1996-07), pages 843-850, XP000857252 New York, NY, USA
- P. HENRICH-NOACK ET AL.: "TGF-beta1 protects hippocampal neurons against degeneration caused by transient global ischemia. Dose-response relationship and potential neuroprotective mechanisms." STROKE, vol. 27, no. 9, September 1996 (1996-09), pages 1609-1614, XP000857240 Dallas, TX, USA

## Description

The present invention relates to pharmaceutical compositions containing a compound capable of substantially inhibiting the biological activity of TGF-β, for treating cerebral disorders and a second compound for disintegrating blood clots.

The transforming growth factor β (TGF-β) family contains subspecies TGF-β1, TGF-β2 and TGF-β3, which are widely distributed and contextually acting cytokines with prominent roles in development and cell cycle control (Roberts and Sporn, 1990; Kriegelstein et al., 1995). TGF-βs have been implicated in the regulation of neuronal survival of e.g. motoneurons, sensory and midbrain dopaminergic neurons. The TGF-β isoforms show widely overlapping expression patterns. Nullmutations for each of these isoforms show distinct phenotypes, which, however, are restricted to non-neural tissues (Shull et al., 1992; Kaartinen et al., 1995; Proetzel et al., 1995; Sanford et al., 1997). Mice deficient for the TGF-β receptor type-II (TβR-II) are lethal at E 10.5, i.e. prior to the development of the nervous system.

During the development of the vertebrate nervous system, large numbers of neurons in the central and peripheral nervous system undergo naturally occuring cell death (Oppenheim, 1991). Regulation of neuron survival or death, respectively, requires the concerted actions of sets of molecules, which act cell-extrinsically and -instrinsically to execute or prevent cell death (Pettmann and Henderson, 1998).

Cerebral disorders such as a neurodegenerative disorder or cerebral ischemiae, result in injury or death of neurons in mammals, and produce motor and/or cognitive deficits that are often permanent. At present, in most of these cerebral disorders there is no treatment that reliably improves the prognosis of a patient suffering from said disorders.

Thus, the technical problem underlying the present invention is to provide a new system imparting protection and therefore survival on predamaged or injured neurons upon a certain cerebral disorder.

The solution of the above technical problem is achieved by providing the embodiments characterized in the claims.

In particular, the present invention is based on the fact that, beside promoting intact neurons, TGF-β is the major regulator for the execution of predamaged neurons upon a certain cerebral disorder. Accordingly, the present invention relates to a pharmaceutical composition containing, in pharmaceutically effective amounts, a compound capable of substantially inhibiting the biological activity of TGF-β, and a second compound for disintegrating blood clots, for treating cerebral disorders in mammals, preferably in man. The term "TGF-β" comprises the subspecies TGF-β1, TGF-β2, and TGF-β3.

in a preferred embodiment of the present invention the term "compound capable of substantially inhibiting the biological activity of TGF-β refers to polyclonal or monoclonal antibodies directed to TGF-β, as TGFG-β inhibitors, or to antagonists directed to TGF-β such as compounds having the binding site of a TGF-β receptor, e.g. a TGF-β RII/Fc chimeric protein (TβR-II=Fc), or to proteinaceous or non-proteinaceous compounds which are capable of, e.g. chemically, altering TGF-β in the organism such that the altered TGF-β is rendered incapable of binding to TGF-β receptors, as well as to low molecular weight compounds such as chemical or non-proteinaceous compounds, as TGF-β-antagonists.

The cerebral disorder includes peripheral and/or CNS-disorders including cerebral and focal ischemiae such as apoplexy, and neurodegenerative disorders such as ALS. For example, adverse consequences of central nervous system injuries may be caused by thrombus, embolus, systemic hypotension, hypertension, hypertensive cerebral vascular disease, rupture of an aneurysm, an angioma, blood dyscrasias, cardiac failure, cardic arrest, cardiogenic shock, septic shock, head trauma, spinal cord trauma, seizure, bleeding from a tumor, or other blood loss. The spinal cord, which is also a part of the central nervous system, is equally susceptible to ischemia resulting from diminished blood flow.

Where the ischemia is associated with a "stroke", it can be either global or focal ischemia, as defined below.

By "focal ischemia", as used herein in reference to the central nervous system, is meant the condition that results from the blockage of a single artery that supplies blood to the brain or spinal cord, resulting in the death of all cellular elements (pan-necrosis) in the territory supplied by that arterty.

By "global ischemia", as used herein in reference to the central nervous system, is meant the condition that results from a general diminution of blood flow to the entire brain, forebrain, or spinal cord, which causes the death of neurons in selectively vulnerable regions throughout these tissues. The pathology in each of these cases is quite different, as are the clinical correlates. Models of focal ischemia apply to patients with focal cerebral infarction, while models of global ischemia are analogous to cardiac arrest, and other causes of systemic hypotension.

A further preferred embodiment of the present invention is a pharmaceutical composition containing, in pharmaceutically effective amounts, the above defined compound, and a second compound for disintegrating blood clots, and optionally a pharmaceutically acceptable carrier and/or diluent. In a preferred embodiment of the present invention, the second compound is selected from the group consisting of urokinase, thrombin, and tPA (tissue plasminogen activator).

The treatment regimen is carried out, in terms of administration mode, timing of the administration, in dosage, so that the functional recovery of the patient from the adverse consequence of the cerebral disorder is improved.

The administration of the compound or pharmaceutical composition according to the present invention can be carried out by any standard route and known rule of administration, including intravenously, orally, or intracerebrally. The dosage of such antibodies or antagonists according to the present invention lies preferably within a range of circulating concentrations that include the ED₅₀ with little or no toxicity. The dosage can vary within this range depending upon the dosage form employed and the route of administration utilized.

The formulation of the above defined pharmaceutical composition according to the present invention does not exhibit any specific restriction, and may be prepared e.g. in the form of tablets, suppositories, solutions, or retarded release-formulations. The antibodies or antagonists for example can be formulated for parenteral administration by injection, for example, by bolus injection or continuous infusion. Formulations for injection can be presented in unit dosage form, for example, in ampules or in multi-dose containers, with an added preservative.

The therapeutic antibodies or antagonists of the invention can also contain a carrier or excipient and/or diluent, many of which are known to skilled artisans. Excipients which can be used include buffers (for example, citrate buffer, phosphate buffer, acetate buffer, and bicarbonate buffer), amino acids, urea, alcohols, ascorbic acid, phospholipids, proteins (for example, serum albumin), EDTA, sodium chloride, liposomes, mannitol, sorbitol, and glycerol.

It is well known in the medical arts that dosages for any one patient depend on many factors, including the general health, sex, weight, body surface area, and age of the patient, as well as the particular compound to be administered, in the time and route of administration, and other drugs being administered concurrently. Determining the most appropriate dosage and route of administration is well within the abilities of a skilled physician.

The following example demonstrates the specifity of anti-TGF-β-antibody as well as morphology and neuron numbers of chick ganglia at E10, i.e. after the main period of ontogenetic ciliary neuron death. Embryos were treated daily from E6 to E9 with 10 *µ*g of a monoclonal mouse anti-TGF-β1 -β2, -β3 antibody (anti-TGF-β; obtained from Genzyme) applied to the chorionic allantoic membrane, an identical dose of a mouse IgG with or without rhodamine conjugation, or 2 *µ*g of a recombinant human TGF-β RII/Fc chimeric protein (TβR-II-Fc; obtained form R&D Systems). Ciliary ganglia were dissected at E10, fixed in Bouin's solution, and paraffin-embedded. Dot blots were prepared showing the specifity of the anti-TGF-β-antibody. Furthermore, sections (H.E. stain or fluorescence microscopy) through the largest circumference of ganglia from embryos were prepared and neuron counts in ganglia with the treatments according to the present invention displayed increased neuron numbers as compared to untreated embryos.

Furthermore, neuron numbers and apoptosis in ciliary ganglia (CG), dorsal root ganglia (L3; DRG), and the lumbar motoneuron column of embryos treated with anti-TGF-β were examined. Neuron counts at E10 following daily treatments from E6 - E9 and TUNEL labelings of CG, DRG, and lumbar motoneurons at E8 following treatment with anti-TGF-β-antibody at E6 and E7 were compared to untreated embryos. A quantitative analysis of TUNEL-positive neurons demonstrated increased or decreased neuron numbers as compared to untreated embryos.

Neuron counts showed a phenotype rescue of CG and motoneurons in anti-TGF-β-treated embryos by administration of TGF-β. A treatment with TGF-β3 (2*µ*g) at E10 following administration of anti-TGF-β from E6 to E9 produced neuron numbers at E14 identical to control embryos. Continuing treatment with anti-TGF-β from E10 to E 13 fully maintained neuron numbers.

Qualitative microscopic and quantitative analyses of H.E. stained sections showed that anti-TGF-β rescues lumbar DRG and motoneurons following unilateral limb bud ablation.

The present invention is illustrated by the following non-limiting example.

### EXAMPLE

### Neuron counting experiments

Chick embryos were treated daily with 50 *µ*l (phosphate buffered saline, supplemented with 200 units/ml peniciflin, 100 *µ*g/ml streptomycin, 200 *µ*g/ml neomycin (each obtained from Gibco) containing either 10 *µ*g of a pan anti-TGF-β (obtained from Genzyme) or 2 *µ*g TβR-II-Fc (obtained from R&D Systems), or 10 *µ*g mouse IgG (obtained from Sigma) by administration onto the chorio-allantoic membrane through a window in the shell as described in Oppenheim et al., 1993. Embryos were killed on either E8, E10 or E14. The CG and the lumbar spinal cord with attached DRG were dissected, fixed in Bouin's solution and paraffin embedded. All tissues were sectioned at 8 to 10 *µ*m and stained with H.E. Neurons were counted in every fifth or tenth section as described in Oppenheim et al., 1993.

### TUNEL labeling

Sections were stained according to the manufacturer's instructions (Boehringer Mannheim) and counter-stained with H.E. TUNEL positive nuclei of large neurons were counted on every tenth section and expressed as ratio of total neuron of this particular neuron population.

### Unilateral limb bud ablation

Ablations of hind limb buds of chick embryos were performed at E3. Embryos were treated daily (10 *µ*g; E3 - E6) as indicated above with either anti-TGF-β or IgG and processed at E7 for H.E. staining and neuron counting.

### Anti-TGF-β-antibody neutralizes endogenous TGF-β during the main period of ontogenetic neuron cell death

In the developing nervous system of chick and mammals, TGF-β2 and -β3 as well as their receptors occur in many populations of postmitotic neurons, including CG, DRG, and motoneurons (Krieglstein et al., 1998). A monoclonal antibody recognizing all three isoforms of TGF-β (anti-TGF-β) was used to neutralize endogenous TGF-β during the main period of ontogenetic cell death (E6 - E9) of CG and DRG as well as spinal motoneurons. Ten *µ*g of this antibody neutralize 10 ng of each available recombinant TGF-β including chicken TGF-β3 (R&D Systems) to > 98%, as assessed in a bioassay using mink lung epithelial cells (Abe et al., 1994; Krieglstein and Unsicker, 1995). The specifity of this monoclonal anti-TGF-β antibody was ascertained by dot blot using 40 other growth factors. Daily treatments with anti-TGF-β resulted in an increased size of CG at E10. A corresponding control IgG, which is rhodamine-conjugated, could be detected throughout the entire embryo. A TβR-II-Fc chimeric protein employed as an alternative tool to capture endogenous TGF-β likewise increased the size of CG at E10 compared to untreated or IgG-treated embryos. Neuron counts revealed that the increased size of CG is due to a significant increase in neuron numbers. The counted values of approximately 6,000 reflect neuron numbers prior to ontogenetic neuron death at E6 (Landmesser and Pilar, 1974) showing that elimination of TGF-β signalling prevents the execution of ontogenetic neuron death in the chick CG.

### Neutralizing TGF-β interferes with ontogenetic cell death in sensory and motoneurons

Sensory and motoneurons were analyzed to investigate whether neutralizing TGF-β also interfered with the development of other neuron populations during the period of ontogenetic neuron death. Neuron counts in the L3 DRG and in the lumbar motoneuron column revealed a significant increase in neuron numbers at E10 following daily antibody treatments from E6 to E9. Numbers of motoneurons in antibody-treated embryos almost matched neuron numbers prior to the onset of ontogenetic neuron death (Hamburger et al., 1975). To determine whether the increase in numbers of neurons upon anti-TGF-β-treatment resulted from reduced ontogenetic neuron death, TUNEL labelling was performed to stain for apoptotic cells. Counting the numbers of TUNEL-positive cells at E8, which is the peak of cell death for all three neuron populations investigated, revealed a significant reduction of the proportion of apoptotic cells. This shows that endogenous TGF-β is essential for executing the cell death program in developing neurons.

### Ontogenetic neuron death occurs after termination of antibody treatment and substitution of endogenous TGF-β

Furthermore, it was investigated whether ontogenetic neuron death would occur at a later time point following termination of the antibody treatment and substitution of endogenous TGF-β. A single dose of TGF-β3 at E10 after the end of the antibody treatment (E6 -E9) resulted in a significant reduction of neuron numbers in the CG and motoneuron column. Numbers of neurons matched those of control embryos at the end of the main ontogenetic cell death period (E10) as well as embryos at E14. Therefore, TGF-β mediates death of selected neurons, especially those that are destined to die. In contrast, prolongation of the antibody treatment beyond the period of ontogenetic death stabilized neuron numbers at increased levels.

### Treatment of limb bud-ablated embryos with anti-TGF-β rescues both motoneurons and DRG neurons

Multiple evidence suggests that the extent of ontogenetic neuron death is crucially regulated by the target (Olek and Edwards, 1978; Pittman and Oppenheim, 1979; Pilar et al., 1980; Thoenen and Edgar, 1985), best exemplified by the classic experiments of V. Hamburger (Hollyday and Hamburger, 1976). Extirpation of the hind limb bud in the E3 chick embryo, i.e. prior to the arrival and synapse formation of motoneuron axons in the target (Dahm and Landmesser, 1988, 1991), causes death of almost all lumbar motoneurons and sensory neurons by E7 (Oppenheim et al., 1978). Qualitative microscopic analysis documented that treatment of limb bud-ablated embryos with anti-TGF-β rescues both motoneurons as well as DRG neurons. Quantification reveals that the lumbar motoneuron population in limb-ablated embryos could surprisingly be rescued by anti-TGF-β treatment to approximately half the half the size as compared to the non-operated control side.

### Summary

The above results show that TGF-β exerts a key role both in the regulation of ontogenetic neuron death of three major classes of peripheral and CNS neurons as well as in the regulation of cell death following target ablation. Parasympathetic CG, sensory DRG, and spinal lumbar motoneurons in chick embroys have their main period of ontogenetic neuron death between E6 and E9, loose approximately 50% of the total cells generated, but require largely distinct and only partly overlapping target-derived trophic molecules to secure the survival of optimal cell numbers. CG neurons are maintained by CNTF/GPA, DRG neurons mainly by neurotrophins, and motoneurons by GDNF, HGF, neurotrophins, IGF-I and others (Nishi, 1994; Heller et al., 1995; Lewin and Barde, 1996; Oppenheim, 1996). In motoneurons, even a cocktail of numerous trophic molecules cannot fully maintain the population. The example of the present invention demonstrates that the elimination of endogenous TGF-β or TGF-β signaling, respectively, results in rescuing effects, which are distinct from neurotrophic factor treatments in terms of (i) the broad spectrum of responsive populations and (ii) magnitude of effect, which encompasses virtually all neurons of a given population. Therefore, the lack of TGF-β is able to rescue all neurons destined to die. The elimination of TGF-β is superior to any of known manipulations of the molecular cascade involved in neuron cell death in that neuron populations are fully maintained.

The example shows that ontogenetic neuron death of ciliary, dorsal root and spinal motoneurons is largely prevented and neuron losses following limb bud ablation are greatly reduced following neutralization of endogenous TGF-β by an anti-TGF-β antibody in chick embryos. Likewise, preventing TGF-β signaling by treatment with a TβR-II fusion protein during the period of ontogenetic cell death in the ciliary ganglion rescues all neurons, which normally die. TUNEL staining revealed decreased numbers of apoptotic cells. Application of exogenous TGF-β rescued the deprived phenotype. Thus, TGF-β in contrast to any single neurotrophic factor acting in the above systems plays a key role in regulating ontogenetic neuron death as well as cell death following neuronal target deprivation.

The above results demonstrate the pivotal role of TGF-β in regulating neuron survival and death. The data given above show that TGF-β is an essential trigger of neuronal death and thus, compounds according to the present invention which are capable of inhibiting the biological activity of TGF-β as e.g. TGF-β-specific antibodies are exceptionally good therapeutic tools that prevent TGF-β signalling in the treatment of stroke, neurotrauma, and neurodegenerative diseases.

### References

Abe et al., *Anal. Biochem. 216,* 276 (1994)
Dahm and Landmesser, *Dev. Biol. 130,* 621 (1988)
Dahm and Landmesser, *J. Neurosci. 11*, 238 (1991)
Flanders et al., *Development 113,* 183 (1991)
Hamburger, *J. Comp. Neurol. 160,* 535 (1975)
Heller et al., *Development 121,* 2681 (1995)
Hollyday and Hamburger, *J. Comp. Neurol*. *170,* 311 (1976)
Kaartinen et al., *Nat. Genet. 11,* 415 (1995)
Krieglstein et al., *Int. J. Dev. Neurosci. 13,* 301 (1995)
Krieglstein and Unsicker, *J. Neurochem. 65,* 1423 (1995)
Krieglstein et al., *J. Neurosci. 18,* 9822 (1998)
Krieglstein et al., *J. Neurobiol. 37,* .563 (1998)
Landmesser and Pilar, *J. Physiol. 247,* 715 (1974)
Lewin and Barde, *Annu. Rev. Neurosci. 19,* 289 (1996)
Nishi, *J. Neurobiol. 25,* 612 (1994)
Olek and Edwards, *Brain Res. 191,* 483 (1978)
Oppenheim et al., *J. Comp. Neurol. 177,* 87 (1978)
Oppenheim, *Annu. Rev. Neurosci. 74,* 453 (1991)
Oppenheim et al., *J. Neurobiol. 24,* 1065 (1993)
Oppenheim, *Neuron 17,* 195 (1996)
Pettmann and Henderson, *Neuron 20,* 633 (1998)
Pilar et al., *J*. *Neurophysiol. 43*, 233 (1980)
Pittman and Oppenheim, 1979
Proetzel et al., *Nat. Genet. 11,* 409 (1995)
Roberts and Sporn, In: *Handbook of Experimental Pharmacology,* M.B. Sporn and A.B. Roberts, eds. (Springer, Heidelberg), Vol. 95, pp. 419-472 (1990)
Sanford et al., *Development 124,* 2659 (1997)
Shull et al., *Nature 359,* 693 (1992)

## Claims

1. A pharmaceutical composition containing, in pharmaceutically effective amounts, a compound capable of inhibiting the biological activity of TGF-β and a second compound for disintegrating blood clots.

2. The pharmaceutical composition according to claim 1, wherein TGF-β comprises TGF-β1, TGF-β2, and/or TGF-β3.

3. The pharmaceutical composition according to claim 1 or 2 further containing a pharmaceutically acceptable carrier and/or diluent.

4. The pharmaceutical composition according to anyone of claims 1 to 3, wherein said compound is an antibody directed to TGF-β or a compound having the binding site of a TGF-β receptor.

5. The pharmaceutical composition according to anyone of claims 1 to 4, wherein said second compound is selected from the group consisting of urokinase and tissue plasminogen activator.

## Patentansprüche

1. Pharmazeutische Zusammensetzung, enthaltend in pharmazeutisch wirksamen Mengen eine Verbindung, die zum Inhibieren der biologischen Aktivität von TGF-β befähigt ist, und eine zweite Verbindung zum Auflösen von Blutgerinnseln.

2. Pharmazeutische Zusammensetzung nach Anspruch 1, wobei TGF-β TGF-β1, TGF-β2 und/oder TGF-β3 umfaßt.

3. Pharmazeutische Zusammensetzung nach Anspruch 1 oder 2, weiter enthaltend einen pharmazeutisch verträglichen Träger und/oder ein pharamzeutisch verträgliches Verdünnungsmittel.

4. Pharmazeutische Zusammensetzung nach einem der Ansprüche 1 bis 3, wobei die Verbindung ein gegen TGF-β gerichteter Antikörper oder eine Verbindung mit einer Bindungsstelle eines TGF-β-Rezeptors ist.

5. Pharmazeutische Zusammensetzung nach einem der Ansprüche 1 bis 4, wobei die zweite Verbindung aus der Gruppe, bestehend aus Urokinase und Gewebsplasminogenaktivator, ausgewählt ist.

## Revendications

1. Composition pharmaceutique contenant, en quantités pharmaceutiquement efficaces, un composé capable d'inhiber l'activité biologique du TGF-β et un second composé pour désintégrer les caillots sanguins.

2. Composition pharmaceutique selon la revendication 1, dans laquelle le TGF-β comprend le TGF-β1, le TGF-β2 et/ou le TGF-β3.

3. Composition pharmaceutique selon la revendication 1 ou la revendication 2, contenant en outre un porteur et/ou un diluant pharmaceutiquement acceptables.

4. Composition pharmaceutique selon l'une quelconque des revendications 1 à 3, dans laquelle ledit composé est un anticorps dirigé contre le TGF-β ou un composé ayant le site de liaison d'un récepteur TGF-β.

5. Composition pharmaceutique selon l'une quelconque des revendications 1 à 4, dans laquelle ledit second composé est choisi dans le groupe comprenant l'urokinase et l'activateur du plasminogène tissulaire.
